# EUROPÄISCHE PATENTSCHRIFT

(11) **EP 3 177 339 B1**
(45) Veröffentlichungstag und Bekanntmachung des Hinweises auf die Patenterteilung: **30.09.2020**
(21) Anmeldenummer: 15750245.1
(22) Anmeldetag: 05.08.2015
(51) Int. Cl.: A61M 1/36

(54) **VERFAHREN ZUM SPÜLEN EINES EXTRAKORPORALEN BLUTKREISLAUFS**
METHOD FOR CLEANING AN EXTRACORPOREAL BLOOD CIRCUIT
PROCÉDÉ DE LAVAGE D'UN CIRCUIT DE SANG EXTRACORPOREL

(30) Priorität: 05.08.2014 DE 102014011671
(43) Veröffentlichungstag der Anmeldung: 14.06.2017
(73) Patentinhaber: Fresenius Medical Care Deutschland GmbH, 61352 Bad Homburg (DE)
(72) Erfinder: WOJKE, Ralf, 61348 Bad Homburg (DE); WIENEKE, Paul, 48149 Münster (DE)
(74) Vertreter: Herrmann, Uwe
(86) Internationale Anmeldenummer: PCT/EP2015/001619
(87) Internationale Veröffentlichungsnummer: WO 2016/020062

(56) Entgegenhaltungen:
- DE-A1- 19 704 564
- MUJAIS S K ET AL: "HEPARIN FREE HEMODIALYSIS USING HEPARIN COATED HEMOPHAN", ASAIO JOURNAL, LIPPINCOTT WILLIAMS & WILKINS / ASAIO, HAGERSTOWN, MD, US, Bd. 42, Nr. 5, 1. September 1996 (1996-09-01), Seiten M538-M541, XP000683623, ISSN: 1058-2916

## Beschreibung

Die Erfindung betrifft ein Verfahren zum Spülen eines extrakorporalen Blutkreislaufs mit einer Spülflüssigkeit sowie eine extrakorporale Blutbehandlungseinheit mit einem extrakorporalen Blutkreislauf und einer Steuereinheit.

Die Vermeidung der Bildung von Blutgerinnseln im extrakorporalen Blutkreislauf ist eine zentrale Aufgabe in der extrakorporalen Blutbehandlung. Generell sind unterschiedliche Lösungskonzepte denkbar.

Eine Möglichkeit ist, dem Patienten ein koagulationshemmendes Mittel wie beispielsweise Heparin zu verabreichen, beispielsweise durch Injektion von Heparin in den extrakorporalen Blutkreislauf. Dies hat den Vorteil einer kontinuierlichen und effizienten Gerinnungshemmung, hat aber unter anderem den Nachteil einer hohen an den Patienten verabreichten Heparin-Dosis.

Des Weiteren besteht die Möglichkeit, den extrakorporalen Blutkreislauf vor Beginn der Behandlung mit einem koagulationshemmenden Mittel zu spülen, das an der Oberfläche des Kreislaufs anhaften soll. Dies hat den Vorteil einer geringen an den Patienten verabreichten Heparin-Dosis, hat aber den Nachteil, dass die Wirkung mit steigender Behandlungsdauer nachlässt und letztlich im Wesentlichen erlischt. Ferner besteht die Möglichkeit, den Kreislauf auch während der Behandlung mit einer Lösung enthaltend ein gerinnungshemmendes Mittel zu spülen. Dies hat den Vorteil einer periodischen Auffrischung der gerinnungshemmenden Wirkung, hat aber den Nachteil einer komplizierten Prozessführung.

Die beiden letztgenannten Alternativen sind verglichen zur Gabe von Heparin an den Patienten aber im Allgemeinen weniger effizient, was die Vermeidung der Bildung von Gerinnseln betrifft. Jedoch wird eine Heparin-freie oder zumindest Heparin-reduzierte Dialysebehandlung aus verschiedenen medizinischen Gründen bei bis zu 7% der Patienten mit akuten Nierenversagen oder mit ESRD angestrebt.

MUJAIS S K ET AL: "HEPARIN FREE HEMODIALYSIS USING HERPARIN COATED HEMOPHAN", ASAIO JOURNAL, LIPPINCOTT WILLIAMS & WILKINS / ASAIO, HAGERSTOWN, MD, US, Bd. 42, Nr. 5, 1. September 1996 (1996-09-01), Seiten M538-M541, offenbart ein Primingverfahren zum Spülen eines extrakorporalen Blutkreislaufs einer Dialysemaschine mit Heparin. DE 197 04 564 A1 betrifft eine Vorrichtung zur periodischen Spülung des extrakorporalen Kreislaufs einer Blutbehandlungseinrichtung.

Der Erfindung liegt die Aufgabe zu Grunde, die Menge an gerinnungshemmenden Mitteln, die an den Patienten systemisch wirksam verabreicht werden, zu verringern und zugleich die Bildung von Gerinnseln im extrakorporalen Blutkreislauf effizient zu vermeiden.

Diese Aufgabe wird durch die Verfahren gemäß der unabhängigen Ansprüche 1 und 2 sowie durch eine extrakorporale Blutbehandlungseinheit gemäß Anspruch 11 gelöst. Weitere vorteilhafte Weiterbildungen der Erfindung sind Gegenstand der Unteransprüche.

Vor diesem Hintergrund betrifft die Erfindung ein Verfahren zum Spülen eines extrakorporalen Blutkreislaufs mit einer Spülflüssigkeit, wobei die Spülflüssigkeit ein gerinnungshemmendes Mittel enthält.

Auf diesem Wege soll das gerinnungshemmende Mittel mit den Schlauchwänden des extrakorporalen Blutkreislaufs und den Membranen des Dialysators kontaktiert werden, sodass es anhaften und die Bildung von Gerinnseln über einen gewissen Behandlungszeitraum verhindern kann.

Bei dem extrakorporalen Blutkreislauf handelt es sich vorzugsweise um den einer Dialysemaschine. Ferner ist ein Einsatz des Verfahrens auch in anderen extrakorporalen Behandlungssystemen denkbar, beispielsweise in Ultrafiltrationsgeräten und Herz-Lungen-Maschinen.

In einer Ausführungsform handelt es sich bei dem gerinnungshemmenden Mittel um Heparin. Heparin ist besonders wirksam und gut verträglich, sodass es in der Medizin sehr häufig eingesetzt wird. Ferner haftet das Heparin, sofern es im Rahmen des erfindungsgemäßen Verfahrens in den extrakorporalen Blutkreislauf eingebracht wird, in ausreichendem Maße an den Schlauchwänden des extrakorporalen Blutkreislaufs und am Dialysator, um über einen gewissen Behandlungszeitraum die Bildung von Gerinnseln zu verhindern.

In einer Ausführungsform wird der extrakorporale Kreislauf vollständig mit der Spülflüssigkeit gefüllt. So kann sichergestellt werden, dass alle Teile des extrakorporalen Blutkreislaufs mit der Spülflüssigkeit und dem gerinnungshemmenden Mittel in Kontakt treten.

In einer Ausführungsform verbleibt die Spülflüssigkeit für eine gewisse Einwirkzeit stationär im Kreislauf. Dies bewirkt ein stärkeres Anhaften bei geringerem Verbrauch als beispielsweise in einem Durchlaufbetrieb. Geeignete Einwirkzeiten betragen bei gängigen Intervallen und Konzentrationen des koagulationshemmenden Mittels beispielsweise zwischen 4 und 15 Minuten oder zwischen 8 und 12 Minuten. In einer Ausführungsform wird die Spülflüssigkeit während der Einwirkzeit bewegt, beispielsweise hin- und her gefördert oder in einem kurzgeschlossenen Kreislauf zirkuliert.

In einer Ausführungsform wird die Spülflüssigkeit nach Ablauf der Einwirkzeit aus dem Kreislauf verdrängt. Dadurch wird verhindert, dass Spülflüssigkeit, die ein koagulationshemmendes Mittel enthält, im weiteren Verlauf in die Blutbahn des Patienten gelangt und dem Patienten so inhärent ein koagulationshemmendes Mittel verabreicht wird. Insoweit kann das Verdrängen mit einer Spülflüssigkeit erfolgen, die frei von dem gerinnungshemmenden Mittel ist. Sofern das Verfahren intermittierend während einer Behandlung durchgeführt wird, kann die Verdrängung auch mit Blut stattfinden.

In einer Ausführungsform werden die Vorgänge des Verbleibens der Spülflüssigkeit im Kreislauf und das anschließende Verdrängen mindestens zweimal und gegebenenfalls mehrmals wiederholt. In diesem Fall kann es sinnvoll sein, das Verdrängen mit einer Spülflüssigkeit durchzuführen, die ebenfalls ein gerinnungshemmendes Mittel, vorzugsweise Heparin enthält. Eine ggf. mehrmalige Wiederholung des Vorganges kann das Ergebnis hinsichtlich der Gerinnungshemmung verbessern.

In einer Ausführungsform verlässt zumindest ein Teil der Spülflüssigkeit den extrakorporalen Kreislauf durch einen Ablauf, der sich vom venösen Port unterscheidet. So kann bei einer intermittierenden Verfahrensführung während der Behandlung die Menge an dem Patienten rückgeführter Spüllösung und die Menge an dem Patienten rückgeführtem koagulationshemmendem Mittel verringert werden.

In einer Ausführungsform umfasst der extrakorporale Blutkreislauf einen Dialysator und der Ablauf entspricht dem Dialysator. Eine Verwendung des Dialysators als Ablauf kann insbesondere dann vorgesehen sein, wenn das Spülverfahren an einem Hämodiafiltrations- oder Hämofiltrationsgerät durchgeführt wird.

In einer Ausführungsform ist der Ablauf in der venösen Leitung angeordnet. Vorzugsweise befindet sich der Ablauf nahe dem venösen Port. Dies vereinfacht eine Verfahrensführung, die nur eine geringe Restmenge der Spüllösung und des koagulationshemmenden Mittels an den Patienten rückführt.

In einer Ausführungsform weist der extrakorporale Blutkreislauf eine venöse Klemme auf, die während des Verfahrens zumindest zeitweise geschlossen ist. Bei dieser Ausführungsform ist der extrakorporale Blutkreislauf nicht mit einem Patienten verbunden.

Ist der extrakorporale Blutkreislauf mit einem Patienten verbunden, ist die Klemme während des Verfahrens geschlossen.

Der Begriff einer Klemme umfasst vorliegend auch ein Sperrventil oder ein Dreiwegeventil. Die Ventile können beispielsweise anhand einer zentralen Steuereinheit mittels Aktoren betrieben werden. Die venöse Klemme ist vorzugsweise zwischen dem Ablauf und dem venösen Port angeordnet. Der Ablauf kann ebenfalls eine Klemme aufweisen, die während des Verfahrens zeitweise geschlossen ist, wobei die Klemme am Anfang des Verfahrens in der Füllphase und am Ende des Verfahrens in der Verdrängungsphase geöffnet sein kann.

In einer Ausführungsform strömt zumindest ein Teil der Spülflüssigkeit durch einen Zulauf in den extrakorporalen Blutkreislauf ein, der sich vom arteriellen Port unterscheidet. Dieser Zulauf kann in der arteriellen Leitung angeordnet sein. In einer Ausführungsform ist der Zulauf stromaufwärts der Blutpumpe angeordnet. Alternativ kann der Zulauf stromabwärts der Blutpumpe angeordnet sein. In einer Ausführungsform werden bestehende Schnittstellen als Zulauf genützt, beispielsweise ein Prädilutions-Port.

In einer Ausführungsform wird die Spülflüssigkeit vor oder nach deren Eintritt in den extrakorporalen Blutkreislauf mit dem gerinnungshemmenden Mittel versetzt. Beispielsweise kann das gerinnungshemmende Mittel aus einem separaten Reservoir der Spülflüssigkeit unmittelbar vor oder bei Eintreten in den extrakorporalen Blutkreislauf zugegeben werden.

In einer Ausführungsform kann das Verfahren vor Beginn der extrakorporalen Blutbehandlung im Rahmen des Primings des extrakorporalen Blutkreislaufs durchgeführt werden, und beispielsweise den abschließenden Schritt des Primingvorgangs darstellen. Prädialytisch kann beispielsweise per Programmsteuerung der extrakorporale Kreislauf mit einer mittels Heparinpumpe heparinisierten Substitutionslösung (evtl. zirkulierend) gefüllt und nach einer vordefinierten Einwirkzeit z.B. mit Dialysat ausgespült. Dieser Vorgang kann ein- oder mehrmals durchgeführt werden. Es entsteht automatisch eine heparinisierte Oberfläche im extrakorporalen Kreislauf.

Alternativ oder zusätzlich ist eine intermittierende Verfahrensführung während der extrakorporalen Blutbehandlung möglich. In diesem Fall kann eine mögliche Verfahrensführung die folgenden Schritte umfassen: (a) Halten der Blutpumpe und Öffnen eines zuvor geschlossenen separaten Zulaufs; (b) vollständiges Befüllen wenigstens eines Abschnitts des extrakorporalen Blutkreislaufs mit einer Spülflüssigkeit, die ein koagulationshemmendes Mittel enthält; (c) Schließen des Zulaufs und einer zuvor offenen venösen Klemme; (d) Abwarten einer Einwirkzeit; (e) Öffnen eines zuvor geschlossenen separaten Ablaufs, der in der venösen Leitung angeordnet ist; (f) Starten der Blutpumpe und Verdrängen der Spülflüssigkeit; (g) Schließen des Ablaufs und Öffnen der venösen Klemme; (h) Fortsetzung der Behandlung. Der Zulauf ist in diesem Fall vorzugsweise stromabwärts der Blutpumpe angeordnet.

Die Erfindung betrifft ferner eine extrakorporale Blutbehandlungseinheit mit einem extrakorporalen Blutkreislauf und einer Steuereinheit, wobei die Steuereinheit ausgebildet ist, um ein erfindungsgemäßes Spülverfahren vor Beginn der Behandlung und/oder intermittierend während der Behandlung durchzuführen.

Das Merkmal, dass die Steuereinheit ausgebildet ist, um ein Verfahren gemäß einem der vorhergehenden Ansprüche durchzuführen, bedeutet, dass in der Steuereinheit eine entsprechende Routine hinterlegt ist und dass die Steuereinheit mit den notwendigen Aktoren (z.B. Pumpen und Ventile) verbunden ist, die für die Durchführung des Verfahrens erforderlich sind.

In einer Ausführungsform handelt es sich bei der extrakorporalen Blutbehandlungseinheit um eine Dialysemaschine. Die Dialysemaschine kann zur Durchführung einer Hämodialyse, einer Hämodiafiltration, einer Hämofiltration und/oder einer Ultrafiltration (reiner Flüssigkeitsentzug) geeignet und bestimmt sein. Ferner kann es sich bei der erfindungsgemäßen extrakorporalen Blutbehandlungseinheit um ein anderes Behandlungssystemen handeln, beispielsweise ein Ultrafiltrationsgerät oder eine Herz-Lungen-Maschine. Die extrakorporale Blutbehandlungseinheit kann bauliche Merkmale umfassen, die im Zusammenhang mit dem erfindungsgemäßen Verfahren beschrieben wurden. So kann der extrakorporale Blutkreislauf beispielsweise einen Ablauf für Spülflüssigkeit aufweisen, der sich vom venösen Port des extrakorporalen Blutkreislaufs unterscheidet.

In einer Ausführungsform ist die Steuereinheit so ausgebildet, dass sie die Behandlung vor Durchführung des intermittierenden Spülverfahrens automatisch unterbricht. Vorzugsweise erfolgen die Auslösung und die Steuerung des Spülverfahrens automatisch anhand der in der Steuereinheit hinterlegten Routine. Eine Unterbrechung kann beispielsweise in Reaktion auf bestimmte Sensormeldungen erfolgen, die auf eine beginnende Verstopfung des Filters schließen lassen.

In einer Ausführungsform ist die Steuereinheit so ausgebildet, dass sie die Behandlung nach Durchführung des intermittierenden Spülverfahrens automatisch fortsetzt.

In einer Ausführungsform ist die Steuereinheit so ausgebildet, dass sie das intermittierende Spülverfahren während der Behandlung in periodischen Zeitabständen aktiviert, wobei die Zeitabstände vorzugsweise zwischen 30 Minuten und 2 Stunden liegen. Erfahrungswerte besagen, dass der gerinnungshemmende Effekt nach vorhergehender Spülung mit einem antikoagulativen Mittel nach spätestens 2 Stunden stark nachlässt, sodass es sinnvoll ist, die Intervalle kleiner als 2 Stunden zu wählen. Gleichzeitig ist eine Unterbrechung der Behandlung in Zeitabständen, die kleiner als 30 Minuten sind, nicht verhältnismäßig.

In einer Ausführungsform ist die Steuereinheit so ausgebildet, dass sie die Menge von durch das Verfahren zugeführter Spülflüssigkeit und/oder durch das Verfahren abgeleiteten Blutes bei der Ermittlung behandlungsspezifischer Parameter, insbesondere bei der Ermittlung des Ultrafiltrationsvolumens berücksichtigt.

Bei der Blutpumpe und/oder der Spülpumpe kann es sich um eine peristaltische Pumpe handeln, beispielsweise um eine vollokkludierende Rollerpumpe. Bei der Spülflüssigkeit kann es sich beispielsweise um eine Substitutions- bzw. Priminglösung handeln. Beispiele umfassen physiologische Kochsalzlösung und Ringer Lösung. Gegebenenfalls kann die Lösung ferner antikoagulative Mittel wie beispielsweise Heparin enthalten.

Weitere Einzelheiten und Vorteile ergeben sich aus den nachfolgend beschriebenen Figuren und Ausführungsbeispielen. In den Figuren zeigen:
- Figur 1:: eine schematische Darstellung von Fluidkreisläufen gemäß einer Dialysemaschine gemäß einer ersten Ausführungsform der Erfindung;
- Figur 2:: eine schematische Darstellung von Fluidkreisläufen gemäß einer Dialysemaschine gemäß einer zweiten Ausführungsform der Erfindung; und
- Figur 3:: eine schematische Darstellung von Fluidkreisläufen gemäß einer Dialysemaschine gemäß einer dritten Ausführungsform der Erfindung.

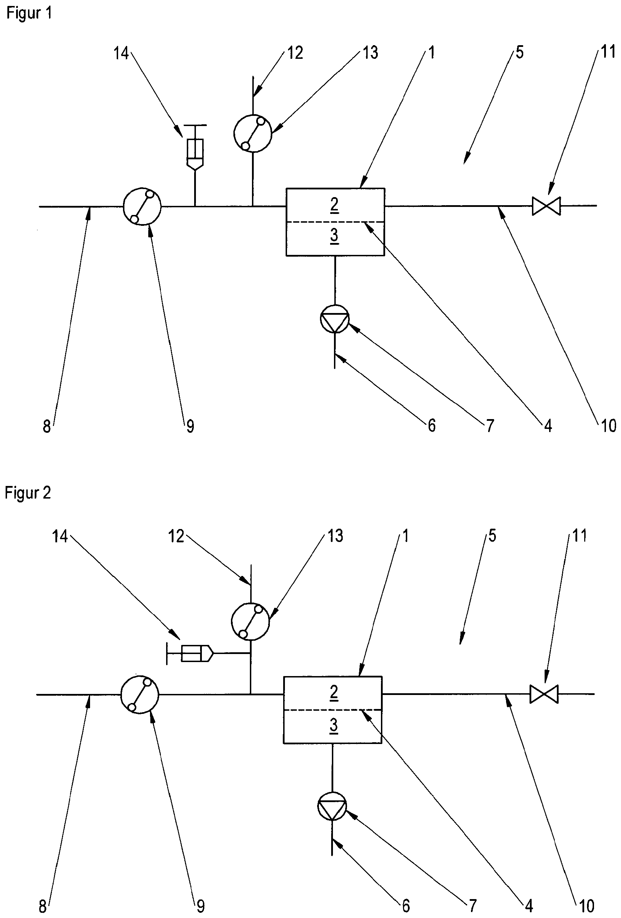

Figur 1 zeigt eine schematische Darstellung von Fluidkreisläufen gemäß einer Dialysemaschine gemäß einer ersten Ausführungsform der Erfindung. Die Dialysemaschine weist einen Dialysator 1 auf, der eine Blutkammer 2 und eine Dialysatkammer 3 aufweist, die durch eine Membran 4 voneinander getrennt sind. Die Blutkammer 2 ist Bestandteil eines extrakorporalen Blutkreislaufs 5. Die Dialysatkammer 3 ist mit einem Ablauf 6 für Dialysat verbunden, in dem eine Ultrafiltrationspumpe 7 angeordnet ist. Etwaige weitere mit der Dialysatkammer verbundene Leitungen wie eine Dialysierflüssigkeitsquelle sind in der Abbildung nicht gezeigt, können aber gleichwohl vorgesehen sein. Der Blutkreislauf 5 weist eine arterielle Leitung 8 mit einer Blutpumpe 9 sowie eine venöse Leitung 10 auf, die mit der Blutseite des Dialysators verbunden sind. Am stromabwärtigen Ende der venösen Leitung befindet sich eine venöse Klemme 11.

In die arterielle Leitung mündet stromabwärts der Blutpumpe eine Prädilutionsleitung 12 mit einer Substitutionspumpe 13. Ferner ist eine Heparinpumpe 14 vorgesehen, die an der arteriellen Leitung zwischen Blutpumpe 9 und Prädilutionsleitung ansetzt. Selbstverständlich ist auch eine andere Positionierung der Prädilutionsleitung und der Heparinpumpe möglich und von der Erfindung umfasst.

Vor Beginn der Behandlung kann ein erfindungsgemäßes Verfahren wie folgt durchgeführt werden. Die arterielle und die venöse Leitung sind nicht mit dem Patienten verbunden. Der extrakorporale Blutkreislauf wird vollständig mit physiologischer Spüllösung gefüllt, beispielsweise unter Verwendung der Prädilutionsleitung oder durch die arterielle oder venöse Leitung, und anschließend werden die arterielle und die venöse Leitung kurzgeschlossen. Anhand der Heparinpumpe wird der im Kreislauf befindlichen Lösung Heparin zugegeben. Anschließend wird die Lösung für 10 Minuten zirkuliert. Nach Beendigung dieses Verfahrensschrittes wird der Kurzschluss gelöst und die heparinhaltige Spüllösung wird durch frische Spüllösung verdrängt, wobei die frische Spüllösung beispielsweise durch die Prädilutionsleitung oder durch die arterielle Leitung in den Kreislauf eindringen kann, und wobei die heparinhaltige Spüllösung beispielsweise durch die venöse Leitung aus dem Kreislauf entfernt werden kann. Gegebenenfalls kann der Vorgang wiederholt werden, d.h. arterielle und venöse Leitung erneut kurzgeschlossen werden, Heparin erneut eingespritzt werden und die Lösung erneut zirkuliert werden.

Figur 2 zeigt eine schematische Darstellung von Fluidkreisläufen gemäß einer Dialysemaschine gemäß einer zweiten Ausführungsform der Erfindung. Bereits aus Figur 1 bekannte Bauteile sind mit entsprechenden Bezugszeichen versehen.

Diese Ausführungsform unterscheidet sich von der in Figur 1 gezeigten Ausführungsform dadurch, dass die Heparinpumpe 14 in die Prädilutionsleitung 12 und nicht in die arterielle Leitung mündet. So kann die Spülflüssigkeit bereits vor Eintreten in den extrakorporalen Blutkreislauf heparinisiert werden.

Anhand dieser Ausführungsform soll nachfolgend ein Beispiel einer intermittierenden Durchführung des erfindungsgemäßen Verfahrens während einer Dialysebehandlung dargestellt werden. Nach periodischen Zeitabständen von 90 Minuten wird während der Behandlung von der Steuereinheit automatisch ein erfindungsgemäßes Spülverfahren durchgeführt. Zu diesem Zweck wird die Substitutionspumpe 13 aktiviert und die Blutpumpe gehalten. Die Spülflüssigkeit wird vor Eintreten in den extrakorporalen Kreislauf die über die Leitung 12 anhand der Heparinpumpe 14 heparinisiert. Sobald der stromabwärts der Mündungsstelle von Leitung 12 befindliche Teil des extrakorporalen Blutkreislaufs mit heparinisierter Spülflüssigkeit gefüllt ist, wird die Substitutionspumpe ebenfalls gehalten, die venöse Klemme 11 geschlossen und die Lösung wird für 10 Minuten stationär im Kreislauf gehalten. Anschließend wird die venöse Klemme geöffnet, die Blutpumpe aktiviert und die Behandlung fortgesetzt. So kann eine lange Einwirkdauer des Heparins bei moderater Heparinabgabe an den Patienten erreicht werden.

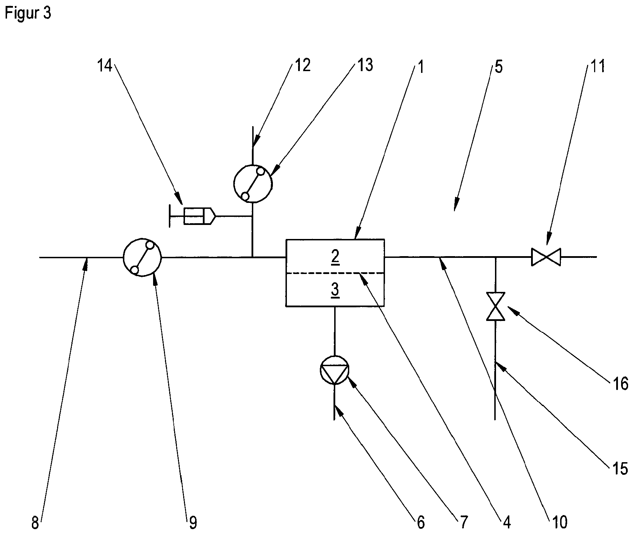

Figur 3 zeigt eine schematische Darstellung von Fluidkreisläufen gemäß einer Dialysemaschine gemäß einer dritten Ausführungsform der Erfindung. Bereits aus Figuren 1 und 2 bekannte Bauteile sind mit entsprechenden Bezugszeichen versehen.

Diese Ausführungsform unterscheidet sich von der in Figur 2 gezeigten Ausführungsform dadurch, dass der extrakorporale Blutkreislauf ferner einen separaten Ablauf 15 für die Spülflüssigkeit aufweist. So wird ermöglicht, dass Spülflüssigkeit nicht über die venöse Leitung, sondern über den separaten Ablauf 15 abfließen kann. Eingangsseitig weist der separate Ablauf 15 eine Klemme 16 auf.

Anhand dieser Ausführungsform kann ein weiteres Beispiel einer intermittierenden Durchführung des erfindungsgemäßen Verfahrens während einer Dialysebehandlung dargestellt werden. Dieses Beispiel unterscheidet sich vom vorangehenden Beispiel dadurch, dass nach der Verweilzeit der heparinisierten Lösung im extrakorporalen Blutkreislauf nicht die venöse Klemme 11 sondern die Ablaufklemme 16 geöffnet wird. Sodann wird die Blutpumpe aktiviert, bis die heparinisierte Spüllösung über den Ablauf 15 aus dem Kreislauf verdrängt wurde und das Blut ungefähr im Bereich der Mündung des Ablaufs angekommen ist. Dies kann anhand geeigneter Sensoren detektiert werden. Anschließend wird die Ablaufklemme 16 geschlossen, die venöse Klemme 11 geöffnet und die Behandlung fortgesetzt. So kann eine lange Einwirkdauer des Heparins bei geringem Heparinverbrauch erreicht werden, und es kann eine Heparinabgabe an den Patienten trotz behandlungsintermittierender Verfahrensführung weitgehend vermieden werden.

Sowohl bei der prä-dialytischen Spülung als auch bei der intra-dialytischen Spülung kann ein separater Ablauf im venösen Zweig hilfreich sein. Normalerweise ist der Ablauf geschlossen (z.B. über eine Klemme in einem Kassettensystem), wird aber automatisch (z.B. druckgesteuert über einen Heparin-Flush-Kontrollalgorithmus) bei Bedarf geöffnet. Ein Teil der heparinisierten Spüllösung wird somit abgeführt. Durch geeignete Steuerung kann somit die heparinisierte Substitutionslösung im arteriellen Zweig, im Dialysator und im venösen Zweig einwirken und über die UF-Pumpe und den Ablauf abgeführt werden. In den Patienten gelangt nur die heparinisierte Substitutionslösung, die nicht durch die UF-Pumpe und den Ablauf entfernt wurde. Die Elemente des Kontrollalgorithmus werden um eine mögliche Ablauf-Klemme erweitert. Eingabeparameter für den Kontrollalgorithmus umfassen so beispielsweise den arteriellen Druck, den venösen Druck sowie den Druck auf der Dialysatseite (Zulauf/Ablauf). Beispielhafte Ausgabeparameter umfassen die Förderrate einer Blut-, Heparin-, Ultrafiltrations- oder Substitutionspumpe, sowie die Steuerung einer venösen, arteriellen oder Ablaufklemme.

Zusammenfassend kann festgehalten werden, dass die Erfindung ein Verfahren und eine Vorrichtung zum vollautomatischen prä-dialytischen Anticlotting-Priming und intra-dialytischen Flushing sowie zur koagulationshemmenden Oberflächenbehandlung des extrakorporalen Kreislaufs bei akuter und chronischer Nierenersatztherapie, z.B. mit HD oder HDF-Verfahren, bereitstellt.

Die Gerinnselbildung in extrakorporalen Kreisläufen und im Speziellen im Rahmen einer heparinarmen oder heparinfreien Dialyse wird verringert oder verhindert. Ein Aspekt der Idee betrifft eine automatisierte zwei-Phasen-Antikoagulierung ohne systemische Flüssigkeits- und Antikoagulanzbelastung des Patienten durch Priming und Flushing. Zum einen wird also ein prädialytisches Spülen des extrakorporalen Kreislaufs mit heparinisierter Lösung und zum anderen - um dem Ermüdungseffekt des Primings entgegenzuwirken - ein intermittierendes Spülen des Kreislaufs mit heparinisierter Lösung bei angehaltener Behandlung vorgeschlagen. Ein weiterer Aspekt besteht darin, dass der extrakorporale Kreislauf vollständig mit der Lösung gefüllt und nach einer bestimmten Einwirkzeit wieder entleert wird. Eine Zirkulation kann in einer Ausführungsform unterbleiben. Ein weiterer Kernaspekt ist die oben näher beschriebene Automatisierung des Verfahrens. Bei einem Aspekt der erfindungsgemäßen Idee wird der Blutfluss stark reduziert oder sogar gestoppt und das Spülvolumen über den Dialysator oder einen zusätzlichen Ablauf abgeführt, sodass der Patient nur marginal (Volumen aus dem Schlauchstück zwischen Ablauf und venösem Anschluss) belastet wird.

## Patentansprüche

1. Verfahren zum Spülen eines extrakorporalen Blutkreislaufs (5), vorzugsweise eines extrakorporalen Blutkreislaufs (5) einer Dialysemaschine, mit einer Spülflüssigkeit, wobei die Spülflüssigkeit ein gerinnungshemmendes Mittel, vorzugsweise Heparin enthält, und wobei die Spülflüssigkeit den extrakorporalen Kreislauf (5) durch einen Ablauf (15) verlässt, der sich vom venösen Port unterscheidet, **dadurch gekennzeichnet, dass** der extrakorporale Blutkreislauf (5) eine venöse Klemme (11) aufweist und diese während des Verfahrens geschlossen ist.

2. Verfahren zum Spülen eines extrakorporalen Blutkreislaufs (5), vorzugsweise eines extrakorporalen Blutkreislaufs (5) einer Dialysemaschine, wobei der extrakorporalen Blutkreislauf nicht mit einem Patienten verbunden ist, mit einer Spülflüssigkeit, wobei die Spülflüssigkeit ein gerinnungshemmendes Mittel, vorzugsweise Heparin enthält, und wobei die Spülflüssigkeit den extrakorporalen Kreislauf (5) durch einen Ablauf (15) verlässt, der sich vom venösen Port unterscheidet, **dadurch gekennzeichnet, dass** der extrakorporale Blutkreislauf (5) eine venöse Klemme (11) aufweist und diese während des Verfahrens zumindest zeitweise geschlossen ist.

3. Verfahren nach Anspruch 1 oder 2, **dadurch gekennzeichnet, dass** der extrakorporale Kreislauf vollständig mit der Spülflüssigkeit gefüllt wird und/oder dass die Spülflüssigkeit für eine gewisse Einwirkzeit stationär im Kreislauf verbleibt und gegebenenfalls bewegt oder in einem kurzgeschlossenen Kreislauf zirkuliert wird.

4. Verfahren nach Anspruch 3, **dadurch gekennzeichnet, dass** die Einwirkzeit zwischen 4 und 15 Minuten, vorzugsweise zwischen 8 und 12 Minuten beträgt.

5. Verfahren nach Anspruch 3 oder 4, **dadurch gekennzeichnet, dass** die Spülflüssigkeit nach Ablauf der Einwirkzeit aus dem Kreislauf verdrängt wird.

6. Verfahren nach Anspruch 5, **dadurch gekennzeichnet, dass** das Verdrängen mit einer Spülflüssigkeit erfolgt, die ebenfalls ein gerinnungshemmendes Mittel, vorzugsweise Heparin enthält, oder dass das Verdrängen mit einer Spülflüssigkeit erfolgt, die frei von dem gerinnungshemmenden Mittel ist.

7. Verfahren nach Anspruch 5 oder 6, **dadurch gekennzeichnet, dass** der Vorgang des Verbleibens der Spülflüssigkeit im Kreislauf und das anschließende Verdrängen mindestens zweimal und gegebenenfalls mehrmalswiederholtwird.

8. Verfahren nach Anspruch 1 oder 2, **dadurch gekennzeichnet, dass** der extrakorporale Blutkreislauf einen Dialysator (1) umfasst und der Ablauf dem Dialysator (1) entspricht, oder dass der Ablauf in der venösen Leitung (10) angeordnet ist.

9. Verfahren nach einem der vorhergehenden Ansprüche, **dadurch gekennzeichnet, dass** die Spülflüssigkeit vor oder nach deren Eintritt in den extrakorporalen Blutkreislauf (5) mit dem gerinnungshemmenden Mittel versetzt wird.

10. Verfahren nach einem der vorhergehenden Ansprüche, **dadurch gekennzeichnet, dass** das Verfahren im Rahmen des Primingvorgangs durchgeführt wird.

11. Extrakorporale Blutbehandlungseinheit, vorzugsweise Dialysemaschine, mit einem extrakorporalen Blutkreislauf (5) und einer Steuereinheit,
**dadurch gekennzeichnet,**
**dass** die Steuereinheit ausgebildet ist, um ein Spülverfahren gemäß einem der vorhergehenden Ansprüche vor Beginn der Behandlung und/oder gemäß Anspruch 1 oder 3-10 in Abhängigkeit von Anspruch 1 intermittierend während der Behandlung durchzuführen.

12. Extrakorporale Blutbehandlungseinheit nach Anspruch 11, **dadurch gekennzeichnet, dass** die Steuereinheit so ausgebildet ist, dass sie die Behandlung vor Durchführung des intermittierenden Spülverfahrens automatisch unterbricht und/oder dass sie die Behandlung nach Durchführung des intermittierenden Spülverfahrens automatisch fortsetzt.

13. Extrakorporale Blutbehandlungseinheit nach Anspruch 11 oder 12, **dadurch gekennzeichnet, dass** die Steuereinheit so ausgebildet ist, dass sie das intermittierende Spülverfahren während der Behandlung in periodischen Zeitabständen aktiviert, wobei die Zeitabstände vorzugsweise zwischen 30 Minuten und 2 Stunden liegen.

14. Extrakorporale Blutbehandlungseinheit nach einem der Ansprüche 11 bis 13, **dadurch gekennzeichnet, dass** die Steuereinheit so ausgebildet ist, dass sie die Menge von durch das Verfahren zugeführter Spülflüssigkeit und/oder durch das Verfahren abgeleiteten Blutes bei der Ermittlung behandlungsspezifischer Parameter, insbesondere bei der Ermittlung des Ultrafiltrationsvolumens berücksichtigt.

## Claims

1. A method of flushing an extracorporeal blood circuit (5), preferably an extracorporeal blood circuit (5) of a dialysis machine, with a flushing liquid, wherein the flushing liquid contains an anticoagulant agent, preferably heparin, and wherein the flushing liquid leaves the extracorporeal circuit (5) through an outflow (15) which differs from the venous port, **characterized in that** the extracorporeal blood circuit (5) has a venous clamp (11) and that the latter is closed during the method.

2. A method of flushing an extracorporeal blood circuit (5), preferably an extracorporeal blood circuit (5) of a dialysis machine, wherein the extracorporeal blood circuit is not connected to a patient, with a flushing liquid, wherein the flushing liquid contains an anticoagulant agent, preferably heparin, and wherein the flushing liquid leaves the extracorporeal circuit (5) through an outflow (15) which differs from the venous port, **characterized in that** the extracorporeal blood circuit (5) has a venous clamp (11) and that the latter is closed at least at times during the method.

3. A method in accordance with claim 1 or claim 2, **characterized in that** the extracorporeal circuit is completely filled with the flushing liquid; and/or **in that** the flushing liquid remains stationary in the circuit for a certain dwell time and is optionally moved or is circulated in a short-circuited circuit.

4. A method in accordance with claim 3, **characterized in that** the dwell time amounts to between 4 and 15 minutes, preferably to between 8 and 12 minutes.

5. A method in accordance with claim 3 or claim 4, **characterized in that** the flushing liquid is displaced from the circuit at the end of the dwell time.

6. A method in accordance with claim 5, **characterized in that** the displacement takes place using a flushing liquid which likewise contains an anticoagulant agent, preferably heparin; or **in that** the displacement takes place using a flushing liquid which is free of the anticoagulant agent.

7. A method in accordance with claim 5 or claim 6, **characterized in that** the process of the remaining of the flushing liquid in the circuit and the subsequent displacement is repeated at least twice and optionally several times.

8. A method in accordance with claim 1 or claim 2, **characterized in that** the extracorporeal blood circuit comprises a dialyzer (1) and the outflow corresponds to the dialyzer (1); or **in that** the outflow is arranged in the venous line (10).

9. A method in accordance with one of the preceding claims, **characterized in that** the flushing liquid is admixed with the anticoagulant agent before or after its entry into the extracorporeal blood circuit (5).

10. A method in accordance with one of the preceding claims, **characterized in that** the method is carried out within the framework of the priming process.

11. An extracorporeal blood treatment unit, preferably a dialysis machine, having an extracorporeal blood circuit (5) and a control unit,
**characterized in that**
the control unit is configured to carry out a flushing method in accordance with one of the preceding claims before the start of the treatment and/or in accordance with claim 1 or claims 3-10 in dependence on claim 1 intermittently during the treatment.

12. An extracorporeal blood treatment unit in accordance with claim 11, **characterized in that** the control unit is configured so that it automatically interrupts the treatment before carrying out the intermittent flushing method; and/or **in that** it automatically continues the treatment after carrying out the intermittent flushing method.

13. An extracorporeal blood treatment unit in accordance with claim 11 or claim 12, **characterized in that** the control unit is configured so that it activates the intermittent flushing method at periodic time intervals during the treatment, with the time intervals preferably lying between 30 minutes and 2 hours.

14. An extracorporeal blood treatment unit in accordance with one of the claims 11 to 13, **characterized in that** the control unit is configured so that it takes account of the quantity of flushing liquid supplied by the method and/or of the blood drained off by the method in the determination of treatment-specific parameters, in particular in the determination of the ultrafiltration volume.

## Revendications

1. Procédé de lavage d'un circuit de sang extracorporel (5), de préférence d'un circuit de sang extracorporel (5) d'une machine de dialyse, au moyen d'un liquide de lavage, le liquide de lavage contenant un anticoagulant, de préférence de l'héparine, et le liquide de lavage quittant le circuit extracorporel (5) par un écoulement (15), qui est différent de la chambre veineuse, **caractérisé en ce que** le circuit de sang extracorporel (5) comporte une pince veineuse (11) et celle-ci est fermée pendant le procédé.

2. Procédé de lavage d'un circuit de sang extracorporel (5), de préférence d'un circuit de sang extracorporel (5) d'une machine de dialyse, le circuit de sang extracorporel n'étant pas relié à un patient, au moyen d'un liquide de lavage, le liquide de lavage contenant un anticoagulant, de préférence de l'héparine, et le liquide de lavage quittant le circuit extracorporel (5) par un écoulement (15), qui est différent de la chambre veineuse, **caractérisé en ce que** le circuit de sang extracorporel (5) comporte une pince veineuse (11) et celle-ci est fermée au moins temporairement pendant le procédé.

3. Procédé selon la revendication 1 ou 2, **caractérisé en ce que** le circuit de sang extracorporel est rempli entièrement avec le liquide de lavage et/ou **en ce que** le liquide de lavage demeure immobile dans le circuit pendant un temps d'action précis et est, le cas échéant, déplacé ou mis en circulation dans un circuit court-circuité.

4. Procédé selon la revendication 3, **caractérisé en ce que** le temps d'action est compris entre 4 et 15 minutes, de préférence entre 8 et 12 minutes.

5. Procédé selon la revendication 3 ou 4, **caractérisé en ce que** le liquide de lavage est expulsé du circuit une fois le temps d'action écoulé.

6. Procédé selon la revendication 5, **caractérisé en ce que** l'expulsion est effectuée avec un liquide de lavage, qui contient également un anticoagulant, de préférence de l'héparine, ou **en ce que** le l'expulsion est effectuée avec un liquide de lavage qui est exempt de l'anticoagulant.

7. Procédé selon la revendication 5 ou 6, **caractérisé en ce que** le processus selon lequel le liquide de lavage demeure dans le circuit et l'expulsion subséquente sont répétés au moins deux fois et le cas échéant plusieurs fois.

8. Procédé selon la revendication 1 ou 2, **caractérisé en ce que** le circuit de sang extracorporel comprend un dialyseur (1) et l'écoulement correspond au dialyseur (1), ou **en ce que** l'écoulement est disposé dans la ligne veineuse (10).

9. Procédé selon l'une des revendications précédentes, **caractérisé en ce que** le liquide de lavage est mélangé avec l'anticoagulant avant ou après son entrée dans le circuit de sang extracorporel (5).

10. Procédé selon l'une des revendications précédentes, **caractérisé en ce que** le procédé est exécuté dans le cadre du processus d'amorçage.

11. Unité extracorporelle de traitement du sang, de préférence machine de dialyse, comprenant un circuit de sang extracorporel (5) et une unité de commande,
**caractérisée en ce que**,
l'unité de commande est conçue pour exécuter un procédé de lavage selon l'une des revendications précédentes avant le début du traitement et/ou selon les revendications 1 ou 3 à 10 en dépendance de la revendication 1, par intermittence pendant le traitement.

12. Unité extracorporelle de traitement du sang selon la revendication 11, **caractérisée en ce que** l'unité de commande est conçue de manière à interrompre automatiquement le traitement avant l'exécution du procédé de lavage intermittent et/ou à poursuivre automatiquement le traitement après l'exécution du procédé de lavage intermittent.

13. Unité extracorporelle de traitement du sang selon la revendication 11 ou 12, **caractérisée en ce que** l'unité de commande est conçue de manière à activer le procédé de lavage intermittent à intervalles réguliers pendant le traitement, les intervalles étant de préférence compris entre 30 minutes et 2 heures.

14. Unité extracorporelle de traitement du sang selon l'une des revendications 11 à 13, **caractérisée en ce que** l'unité de commande est conçue de manière à prendre en compte la quantité de liquide de lavage alimenté par le procédé et/ou de sang évacué par le procédé lors de la détermination de paramètres spécifiques du traitement, en particulier lors de la détermination du volume d'ultrafiltration.
